**Europäisches Patentamt**

**European Patent Office**

**Office européen des brevets**

(11) Veröffentlichungsnummer: **0 416 484 A1**

## (12) EUROPÄISCHE PATENTANMELDUNG

(21) Anmeldenummer: 90116805.4

(22) Anmeldetag: 01.09.90

(51) Int. Cl.⁵: **C12N 9/96**, C12Q 1/56, //A61K35/14

(30) Priorität: 04.09.89 DE 3929329

(43) Veröffentlichungstag der Anmeldung:
13.03.91 Patentblatt 91/11

(84) Benannte Vertragsstaaten:
AT BE CH DE ES FR GB IT LI LU NL SE

(71) Anmelder: **BEHRINGWERKE Aktiengesellschaft**
**Postfach 1140**
**W-3550 Marburg 1(DE)**

(72) Erfinder: **Stief, Thomas, Dr.**
**Avda. Kansas City 28, Bloque 1, Ap. 225**
**E-41007 Sevilla(ES)**
Erfinder: **Heimburger, Norbert, Prof. Dr.**
**Sonnenhang 10**
**W-3550 Marburg(DE)**

(74) Vertreter: **Klein, Otto, Dr. et al**
**Hoechst AG Zentrale Patentabteilung**
**Postfach 80 03 20**
**W-6230 Frankfurt am Main 80(DE)**

(54) **Stabilisierungsmittel zur Verbesserung der Wiederfindung von Gerinnungsfaktoren in Blutproben.**

(57) Die vorliegende Erfindung bezieht sich auf ein Mittel zur Stabilisierung der funktionellen Aktivitäten von Gerinnungsfaktoren in Proben biologischer Flüssigkeiten, insbesondere auf die Stabilisierung von Plasminogen-Aktivatoren.

EP 0 416 484 A1

## STABILISIERUNGSMITTEL ZUR VERBESSERUNG DER WIEDERFINDUNG VON GERINNUNGSFAKTOREN IN BLUTPROBEN

Die vorliegende Erfindung bezieht sich auf ein Mittel zur Stabilisierung der funktionellen Aktivitäten von Gerinnungsfaktoren in Proben biologischer Flüssigkeiten, insbesondere auf die Stabilisierung von Plasminogen-Aktivatoren.

Der funktionelle Nachweis der Aktivität von Gerinnungsfaktoren ist von großer klinischer Bedeutung, sowohl hinsichtlich Therapie als auch bezüglich der Diagnostik.

Aktivitätsnachweise von Gerinnungsfaktoren gestalten sich jedoch schwierig, da im Blut vorhandene spezifische und unspezifische Inhibitoren die Aktivitäten in vitro irreversibel in Abhängigkeit der Standzeit der entnommenen Blutprobe unterdrücken.

So ist z. B. der funktionelle Nachweis von Plasminogen-Aktivatoren (PA), einerseits vom urinären (u-PA), andererseits vom Gewebe (t-PA)-Typ von großer klinischer Bedeutung, sowohl hinsichtlich der Überwachung einer Fibrinolysetherapie als auch bezüglich der Diagnostik einer Disposition zu thrombotischen Ereignissen.

Selbst bei minimalem Zeitaufwand, d. h. auch nach der kürzesten Zeit, die für Probeentnahme und -aufarbeitung (Zentrifugation) erforderlich ist, kann bis zu 80 - 90 % der PA-Aktivität in vitro verlorengehen.

Die der vorliegenden Erfindung zugrundeliegende Aufgabe bestand nun darin, ein Mittel zu finden, welches sowohl als Probeentnahme- als auch als Inkubationsmedium verwendet werden kann, das die Aktivität der Gerinnungsfaktoren über den für eine zuverlässige Bestimmung der Gerinnungsfaktoren notwendigen Zeitraum stabilisiert. Überraschenderweise wurde nun gefunden, daß ein Medium, das neben der Probe mindestens entweder eine Diaminomonocarbonsäure, eine chaotropisch wirksame Substanz, ein Oxidationsmittel oder eine Kombination von mindestens zwei dieser Verbindungen, in Verbindung mit einem der üblichen Antikoagulantien, wie z. B. EDTA oder Citrat enthält, eine nahezu 100%ige Wiederfindung von PA-Aktivität nach ca. 1 Stunde Standzeit bei Raumtemperatur ermöglicht.

Die Erfindung betrifft nun ein Mittel zur Stabilisierung funktioneller Aktivität von Gerinnungsfaktoren in Proben biologischer Flüssigkeiten, wobei das Mittel, neben einem der üblichen Antikoagulantien, wie z. B. EDTA oder Citrat, mindestens eine Verbindung aus einer der folgenden Substanzklassen enthält:

(I) Diaminomonocarbonsäuren

(II) Chaotropika

(III) Oxidationsmittel.

Bevorzugt ist dabei die Verwendung eines Oxidationsmittels, welches bei pH 7.5 - 9 vorzugsweise Methionin in Methioninsulfoxid umwandelt.

Besonders bevorzugt ist die Verwendung von Arginin als Diaminomonocarbonsäure, CsCl als Chaotropikum und Chloramin oder eines seiner Derivate sowie HOCl als Oxidationsmittel. Die Argininkonzentration im Ansatz sollte dabei 25 - 150, vorzugsweise 40 - 80 mmol/l, die CsCl-Konzentration 50 - 200, vorzugsweise 80 - 150 mmol/l, und die Chloramin-Konzentration 1 - 20, vorzugsweise 2 - 10 mmol/l betragen.

Weiterhin bevorzugt ist die Verwendung von Arginin und Chloraminen sowie einer Kombination dieser Stoffe.

Ganz besonders bevorzugt ist die Verwendung des Mediums zur Stabilisierung der Plasminogen-Aktivator-Aktivität.

Ferner betrifft die Erfindung die Verwendung des Mediums als Probenahmemedium, wobei bevorzugterweise ein Teil einer zehnfach konzentrierten Lösung vorgelegt wird und mit 9 - 10 Teilen Patientenblut aufgefüllt wird.

Die Erfindung betrifft außerdem ein Verfahren zur Bestimmung von PA, wobei die Probe entweder schon in dem erfindungsgemäßen Medium aufgenommen worden ist, oder das, mit einem Antikoagulans versetzte Blut mit dem erfindungsgemäßen Medium versetzt wird, bevor es auf die PA-Aktivität untersucht wird. Die Bestimmung der PA-Aktivität kann dabei grundsätzlich nach einem der dem Fachmann bekannten Verfahren erfolgen. Bevorzugt ist die Verwendung des in der EP-A-0 297 597 beschriebenen Verfahrens.

Die folgenden Beispiele beschreiben spezielle Ausführungsformen der Erfindung und sollen die Erfindung in keiner Weise einschränken.

Beispiel 1

Wiederfindung von u-PA in Blut und Plasma

2 ml Citratblut wurden mit 200 µl Substanz (siehe Tabelle 1) versetzt. Daraufhin wurden 100 IE u-PA oder zur Kontrolle physiologische NaCl-Lösung zugesetzt, 60 min bei Raumtemperatur inkubiert, 20 min bei 2000 x g abzentrifugiert und das Plasma nach der von Stief et al. in der EP-A-0 297 597 beschriebenen Methode auf PA-Aktivität untersucht. Hierzu wurden 50 µl Plasma mit 200 µl Plasminogen (15 CTA-U/ml in: 100 mmol/l TRIS, 100 mmol/l NaCl, 1 % Polygeline, 0.1 % Triton X100, pH 8.4 (TNPT-Puffer)) und 200µl 8 mmol/l Chloramin T, 3 mmol/l Tranexamsäure versetzt und 5 min bei 37° C inkubiert. Daraufhin wurde 500 µl 0.6 mmol/l Nva-CHA-Lys-pNA in 480 mmol/l NaCl, 50 mmol/l CsCl (CS-Lösung) zugegeben, 2 min bei 37° C inkubiert, die Reaktion mit 100 µl 8.4 mol/l Essigsäure abgestoppt und die entstandene Extinktion bei 405 nm bestimmt.

Nullwertabgleich: Plasminogenlösung wurde ersetzt durch TNPT-Puffer.

Ergebnis siehe Tabelle 1.

Tabelle 1

| Zusatz | gemessene Plasmin-Aktivität (A) | Bemerkungen |
|---|---|---|
| 1. phys. NaCl-Lösung | 0.153 | |
| 2. ZnCl₂ 0,1 mmol/l | 0.161 | leicht hämolyt. |
| 3. ZnCl₂ 0,2 mmol/l | 0.137 | hämolytisch |
| 4. CsCl 100 mmol/l | 0.491 | |
| 5. CsCl 50 mmol/l | 0.353 | |
| 6. KCl 200 mmol/l | 0.362 | |
| 7. NaCl 240 mmol/l | 0.141 | leicht hämolyt. |
| 8. Chloramin T 5 mmol/l | 1.216 | |
| 9. Chloramin T 10 mmol/l | 1.312 | |
| 10.Chloramin T 20 mmol/l | 1.524 | leicht hämolyt. |
| 11. Arginin 60 mmol/l | 1.130 | |
| 12. Arginin 200 mmol/l | 0.325 | |

Man erkennt, daß u-PA-Aktivität bei Zusatz von Chloramin T, Arginin oder CsCl zu Citratblut zu einem hohen Maße wiedergefunden wird, während beispielsweise Zusatz von physiol. NaCl-Lösung zu einem nahezu völligen Verlust der PA-Aktivität führt. Zugabe von Chloraminen über 10 mmol/l resultiert allerdings in Hämolyseneigung der Bluterythrozyten.

Beispiel 2

Wiederfindung von u-PA in Blut und Plasma in Abhängigkeit von der Standzeit

Testdurchführung gemäß Beispiel 1, u-PA wurde diesmal einerseits zu Blut, andererseits zu Plasma gegeben und die Proben eine bzw. zwei Stunden stehengelassen. 100 % Wiederfindungswert: Zusatz von u-PA zu normalem Plasma, anschließend direkte PA-Aktivitätstestung. Ergebnis siehe Tabelle 2.

EP 0 416 484 A1

Tabelle 2

| Zusatz | Plasminaktivität (A) nach Lagerung bei RT | | % Abfall/h |
|---|---|---|---|
| | 1 Std. | 2 Std. | (%) |
| a) Blut | | | |
| 1. phys. NaCl | 0.154 | 0.093 | 40 |
| 2. CsCl 100 mmol/l | 0.624 | 0.472 | 25 |
| 3. CsCl 200 mmol/l | 0.821 | 0.671 | 18 |
| 4. Chloramin T 5 mmol/l | 1.341 | 1.029 | 23 |
| 5. Arginin 60 mmol/l | 1.234 | 1.118 | 9 |
| 6. Arginin 120 mmol/l | 1.166 | 1.046 | 19 |
| 7. Arginin + CsCl (60/100 mmol/l) | 1.395 | 1.236 | 11 |
| b) Plasma | | | |
| 1. phys. NaCl | 0.093 | 0.057 | 40 |
| 2. CsCl 100 mmol/l | 0.262 | 0.175 | 33 |
| 3. CsCl 200 mmol/l | 0.382 | 0.288 | 25 |
| 4. Chloramin T 5 mmol/l | 0.764 | 0.689 | 10 |
| 5. Arginin 60 mmol/l | 0.804 | 0.749 | 7 |
| 6. Arginin 120 mmol/l | 0.832 | 0.810 | 3 |
| 7. Arginin + CsCl (60/100 mmol/l) | 0.866 | 0.862 | 1 |
| 100 % Kontrollwert: 0.884 | | | |

Man erkennt, daß Arginin, Chloramin und CsCl, insbesondere auch die Kombination aus Arginin und CsCl zu einer stark erhöhten Wiederfindungs- und abgeschwächten Abfallrate von PA-Aktivität in Blut und Plasma führen.

Beispiel 3

Wiederfindung von t-PA-Aktivität in Vollblut und Plasma

2 ml Portionen Vollblut und Plasma I wurden zunächst mit 200 $\mu$l unterschiedlicher Substanzen, später mit 200 IE/ml Einketten-t-PA oder physiol. NaCl (Kontrolle) versetzt, 1 Stunde bei Raumtemperatur stehengelassen und das Blut 20 min bei 2000 x g abzentrifugiert (= Plasma II). 50 $\mu$l Proben von Plasma I und Plasma II wurden auf deren t-PA-Aktivität untersucht, indem 50 $\mu$l Probe mit 200 $\mu$l Plasminogen (3 CTA-U in TNPT-Puffer), 100 $\mu$l 16 mmol/l Chloramin T und 100 $\mu$l Fibrinogen-Spaltprodukte (200 $\mu$g, erhalten gemäß Stief et al., Thromb.Res. 48 , 603, 1987) oder 100 $\mu$l TNPT-Puffer zum Nullwertabgleich 10 min bei 37$^\circ$ C inkubiert wurden, 500 $\mu$l $\overline{CS}$-Lösung zugesetzt, nochmals 5 min bei 37$^\circ$ C inkubiert und die Substratumsetzung durch Zusatz von 100 $\mu$l 8,5 mol/l Essigsäure beendet wurde. 100%-Kontrollwert: t-PA-Zugabe zu Plasma, sofortige Messung. Ergebnis siehe Tabelle 3.

4

Tabelle 3

| Zusatz | Plasminaktivität (A) in | |
|---|---|---|
| | a) Plasma I | b) Plasma II (Vollblut) |
| phys. NaCl | 0.087 | 0.110 |
| CsCl 100 mmol/l | 0.103 | 0.212 |
| Chloramin T (CT)5 mmol/l | 0.309 | 0.658 |
| Arginin (Arg)60 mmol/l | 0.201 | 0.575 |
| 100 % - Kontrollwert: 0.332 | | |

Man erkennt, daß Zusatz von Chloramin T bzw. Arginin zu erheblich verbesserten Wiederfindungsraten von PA-Aktivität führt.

Beispiel 4

Wiederfindung von PA-Aktivität in Plasma, Einfluß von Kombinationen

2 ml Portionen Plasma wurden mit 200 $\mu$l unterschiedlicher Substanzen mit 40 IE/ml u-PA, bzw. 200 IE/ml Einketten-t-PA versetzt, die PA-Aktivität sofort und nach einer Stunde gemäß Beispiel 1 (für u-PA) und Beispiel 2 (für t-PA) bestimmt. Endinkubation bei u-PA: 2 1/4 min, t-PA> 10 min. Ergebnis siehe Tabelle 4.

Tabelle 4

| Zusatz | u-PA Wiederfindung | t-PA-Wiederfindung |
|---|---|---|
| | Plasminaktivität (A) nach 1 h 37° C (% der Ausgangsaktivität) | |
| phys. NaCl | 21 | 70 |
| CsCl 100 mmol/l | 48 | 72 |
| CT 5 mmol/l | 103 | 100 |
| Arg 60 mmol/l | 104 | 100 |
| CsCl + CT (100/5 mmol/l) | 106 | 82 |
| CsCl + Arg (100/60 mmol/l) | 100 | 110 |
| CT + Arg (5/60 mmol/l) | 110 | 115 |
| CsCl + CT + Arg (100/5/60 mmol/l) | 110 | 100 |

Vorzugsweise Arginin und Chloramin T eignen sich, um PA-Aktivitäten quantitativ auch nach längerer Probenstandzeit nachzuweisen.

**Ansprüche**

1. Mittel zur Stabilisierung funktioneller Aktivität von Gerinnungsfaktoren in Proben biologischer Flüssigkeiten, dadurch gekennzeichnet, daß das Mittel, neben einem der üblichen Antikoagulantien, wie z. B. EDTA oder Citrat, mindestens eine Verbindung aus einer der folgenden Substanzklassen enthält:

(I) Diaminomonocarbonsäuren

(II) Chaotropika

(III) Oxidationsmittel.

2. Mittel gemäß Anspruch 1, gekennzeichnet durch die Verwendung eines Oxidationsmittels, welches bei pH 7.5-9 vorzugsweise Methionin in Methioninsulfoxid umwandelt.

3. Mittel gemäß Anspruch 1, dadurch gekennzeichnet, daß Arginin als Diaminomonocarbonsäure, CsCl als Chaotropikum und Chloramin oder eines seiner Derivate sowie HOCl als Oxidationsmittel eingesetzt werden und die Argininkonzentration im Ansatz, nach Zugabe der Probe, 25 - 150, vorzugsweise 40 - 80 mmol/l, die CsCl-Konzentration 50 - 200, vorzugsweise 80 - 150 mmol/l, und die Chloraminkonzentration 1 - 20, vorzugsweise 2 - 10 mmol/l betragen.

4. Mittel gemäß Anspruch 1 zur Stabilisierung der Plasminogen-Aktivator Aktivität.

5. Methode zur Blutentnahme, dadurch gekennzeichnet, daß das Blut in einem Gefäß aufgefangen wird, in dem ein solches Volumen einer mehrfach konzentrierten Lösung des Mittels gemäß Anspruch 1 vorgelegt wird, daß nach der Blutentnahme die gewünschte Konzentration der Wirkstoffe des Mittels erreicht wird.

6. Verfahren zur Bestimmung von PA, wobei die Probe entweder schon in dem Mittel gemäß Anspruch 1 aufgenommen worden ist, oder das, mit einem Antikoagulans versetzte Blut mit dem Mittel gemäß Anspruch 1 versetzt wird, bevor es auf die PA-Aktivität untersucht wird.

Patentansprüche für folgenden Vertragsstaat: ES

1. Verfahren zur Herstellung eines Mittels zur Stabilisierung der funktionellen Aktivität von Gerinnungsfaktoren in Proben biologischer Flüssigkeiten, dadurch gekennzeichnet, daß ein übliches Antikoagulants, vorzugsweise EDTA oder Citrat, mit mindestens einer Verbindung aus einer der folgenden Substanzklassen der

(I) Diaminomonocarbonsäuren,

(II) Chaotropika oder

(III) Oxidationsmittel

zusammengebracht wird.

2. Verfahren gemäß Anspruch 1, gekennzeichnet durch die Verwendung eines Oxidationsmittels, welches bei pH 7.5-9 Methionin in Methioninsulfoxid umwandelt.

3. Mittel gemäß Anspruch 1, dadurch gekennzeichnet, daß Arginin als Diaminomonocarbonsäure, CsCl als Chaotropikum und Chloramin oder eines seiner Derivate sowie HOCl als Oxidationsmittel eingesetzt werden und die Argininkonzentration im Ansatz, nach Zugabe der Probe, 25 - 150, vorzugsweise 40 - 80 mmol/l, die CsCl-Konzentration 50 - 200, vorzugsweise 80 - 150 mmol/l, und die Chloramin-Konzentration 1 - 20, vorzugsweise 2 -mmol/l betragen.

4. Verfahren zur Erhaltung der Aktivität von Gerinnungsfaktoren in entnommenem Blut, dadurch gekennzeichnet, daß das Blut in einem Gefäß aufgefangen wird, in dem ein solches Volumen einer mehrfach konzentrierten Lösung eines gemäß Anspruch 1 hergestellten Mittels vorgelegt wird, daß in dem entnommenen Blut die gewünschte Konzentration der Wirkstoffe des Mittels erreicht wird.

5. Verfahren zur Bestimmung von Plasminogen-Aktivator (PA), wobei die Probe entweder schon in einem gemäß Anspruch 1 hergestellten Mittel aufgenommen worden ist, oder wobei das mit einem Antikoagulans versetzte Blut mit dem gemäß Anspruch 1 hergestellten Mittel versetzt wird, bevor es auf die PA-Aktivität untersucht wird.

| Europäisches Patentamt | EUROPÄISCHER RECHERCHENBERICHT | Nummer der Anmeldung |
|---|---|---|
| | | EP 90 11 6805 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 297 597 (BEHRINGWERKE AG)<br>* Das ganze Dokument *<br>– – – | 1-6 | C 12 N 9/96<br>C 12 Q 1/56 //<br>A 61 K 35/14 |
| A | BIOCHEMISTRY, Band 25, Nr. 21, 21. Oktober 1986, Seiten 6351-6355, American Chemical Society, Easton, US; D.A. LAWRENCE et al.: "Inactivation of plasminogen activator inhibitor by oxidants"<br>* Das ganze Dokument *<br>– – – | 1-3 | |
| A | THE JOURNAL OF BIOLOGICAL CHEMISTRY, Band 262, nr. 13, 5. Mai 1987, Seiten 6055-6059, The American Society of Biological Chemists, Inc., Baltimore, US; B.-H. SHIEH et al.: "The reactive site of human alpha2-antiplasmin"<br>– – – – – | | |

| | RECHERCHIERTE SACHGEBIETE (Int. Cl.5) |
|---|---|
| | C 12 Q 1/00<br>G 01 N 33/00<br>A 61 K 35/00<br>A 61 B 5/00 |

Der vorliegende Recherchenbericht wurde für alle Patentansprüche erstellt

| Recherchenort | Abschlußdatum der Recherche | Prüfer |
|---|---|---|
| Den Haag | 30 Oktober 90 | DOEPFER K-P. |